# FASCICULE DE BREVET EUROPEEN

(11) **EP 0 649 297 B1**
(45) Date de publication et mention de la délivrance du brevet: **01.09.1999**
(21) Numéro de dépôt: 94909169.8
(22) Date de dépôt: 09.03.1994
(51) Int. Cl.: A61F 2/38

(54) **IMPLANT TIBIAL POUR PROTHESE DU GENOU**
TIBIALIMPLANTAT FÜR KNIEPROTHESE
TIBIAL IMPLANT FOR A KNEE PROSTHESIS

(30) Priorité: 10.03.1993 FR 9303034
(43) Date de publication de la demande: 26.04.1995
(73) Titulaire: MEDINOV S.A., F-42312 Roanne (FR)
(72) Inventeur: DELFOSSE, Jacques, F-54000 Nancy (FR)
(74) Mandataire: Martin, Jean-Paul
(86) Numéro de dépôt international: FR9400260
(87) Numéro de publication internationale: WO9420047

(56) Documents cités:
- FR-A- 2 076 838
- FR-A- 2 653 992
- US-A- 4 822 362
- US-A- 4 944 757

## Description

L'invention concerne plus particulièrement une prothèse tricompartimentale à glissement du genou.

De manière parfaitement connue, ce type de prothèse comprend essentiellement un implant fémoral et un implant tibial. L'invention concerne essentiellement un implant tibial du type de ceux comprenant une embase métallique recevant un plateau en polyéthylène, convenablement profilé, pour recevoir, à glissement, les patins condyliens de l'élément fémoral.

Très souvent, les embases tibiales présentent une quille de stabilité et/ou d'ancrage destinée à être impactée dans le tissu spongieux de l'extremité proximale du tibia Ces quilles sont solidaires directement ou de manière rapportée de l'embase tibiale et peuvent présenter des allettes de stabilité et faisant office d'éléments anti-rotatoires.

Le FR-A-2 653 992 décrit une embase tibiale équipée de bossages traversés par des quilles d'ancrage tibial.

Dans le cas de quilles rapportées, ces dernières sont le plus souvent fixées au moyen de vis, sous l'embase tibiale. Cet état de la technique peut être illustré à titre indicatif nullement limitatif, par l'enseignement des brevets FR 9103595, FR 9109247, US 4938769, US 4944757.

Quelles que soient les solutions techniques enseignées par ces brevets, la quille est positionnée en appui contre la face de dessous de l'embase tibiale, tandis qu'une ou des vis sont engagées à partir de la face de dessus de ladite embase, dans des agencements correspondants de la quille. Eventuellement, la quille peut présenter une tête de centrage, engagée dans une ouverture de l'embase tibiale ; c'est le cas par exemple, des brevets précités FR 9109247 et US 4944757.

Là encore, il est obligatoire d'engager la quille par le dessous de l'embase et ensuite d'assurer sa fixation par le dessus.

Il apparait donc que, dans tous les cas, la fixation de la quille s effectue par le dessous de l'embase tibiale, de sorte qu'il est nécessaire de rendre solidaire la quille à l'embase, avant son impaction.

De telles dispositions ne sont pas rationnelles au niveau de la qualité de la fixation, étant donné que c est la quille qui impose le positionnement de l'embase par rapport à l'extrémité proximale du tibia, de sorte que les appuis ne sont pas respectés.

L'invention s'est fixée pour but de remédier à ces inconvénients, de manière simple, sûre, efficace et rationnelle.

Le problème que se propose de résoudre l'invention est de pouvoir positionner et impacter l'embase tibiale en respectant les différentes coupes et appuis préalablement déterminés, puis, dans un deuxième temps seulement, mettre en place, si nécessaire, une quille de stabilité et/ou d'ancrage.

Pour résoudre un tel problème, il a été conçu et mis au point une embase tibiale conforme à la revendication 1.

Pour résoudre le problème posé d'assurer la fixation primaire de l'embase tibiale avant engagement de la quille de stabilité, l'empreinte est prolongée par un bossage débordant de la face de dessous de l'embase tibiale, ledit bossage étant profilé pour faire office de pré-quille d'ancrage.

Avantageusement, le bossage présente une extrémité tronconique.

Pour résoudre le problème posé d'assurer le blocage en hauteur de la quille, compte-tenu de son engagement à partir de la face de dessus de l'embase, la quille d'ancrage rapportée présente une tête épaulée apte à coopérer en appui dans un chambrage de l'empreinte.

L'empreinte et la tête sont de forme générale circulaire.

Dans le cas d'une quille à ailettes destinées à assurer la stabilité rotatoire, l'empreinte présente des fentes débouchantes dont le nombre et l'orientation correspondent au nombre et à l'orientation des ailettes, de la quille en vue de leur engagement.

Suivant une autre caractéristique de l'invention, la tête de la quille présente des agencements de fixation avec l'empreinte.

Les agencements sont constitués par des fentes verticales formées à partir de la périphérie de la tête, en vue de son expansion diamètrale sous l'action d'une vis de serrage.

Pour résoudre le problème posé d'assurer la fixation de l'embase équipée ou non de la quille au niveau du spongieux et/ou des corticales périphériques tibiales, l'embase tibiale présente des trous pour le passage de vis de fixation.

Le problème posé d'assurer la mise en place du plateau tibial après fixation de l'embase est résolu en ce que l'embase tibiale est asymétrique et présente un rebord périphérique agencé pour la fixation du plateau tibial en polyéthylène.

L'invention est exposée, ci-après plus en détail à l'aide des dessins annexés, dans lesquels :

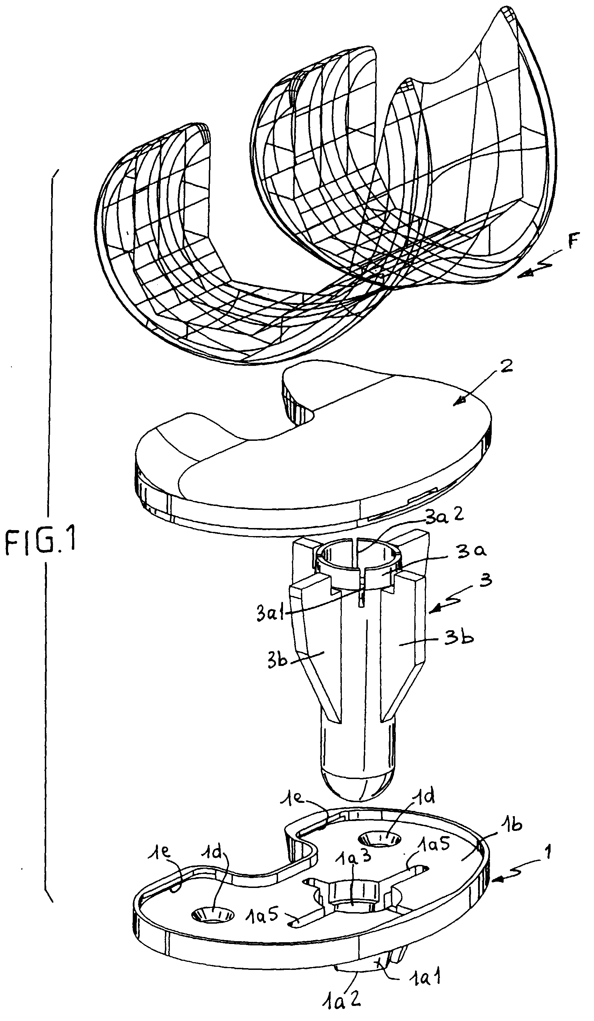

La figure 1 est une vue en perspective montrant les principaux éléments de l'implant tibial qui sont représentés alignés en ordre de montage ; on a également illustré un exemple d'un implant fémoral.

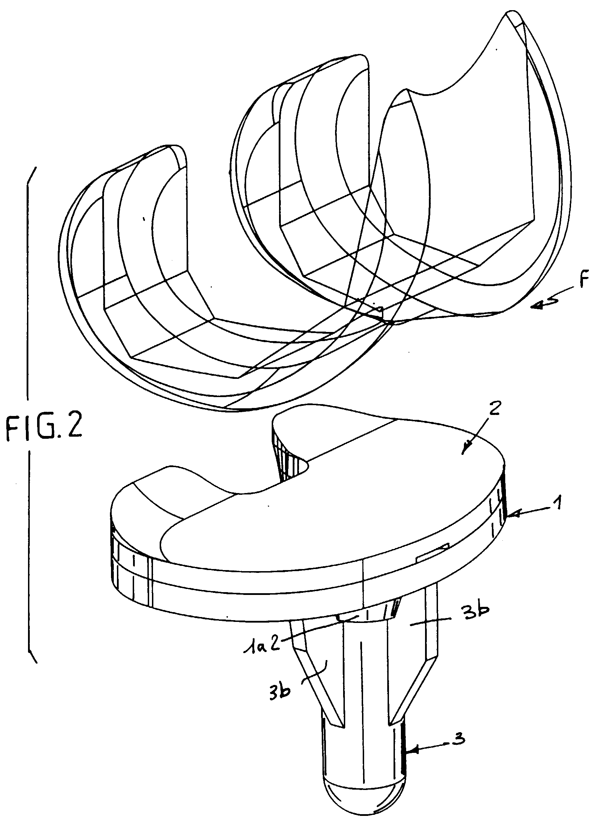

La figure 2 est une vue en perspective correspondant à la figure 1, après montage des éléments de l'implant tibial qui est représenté de trois quarts vue de dessus.
La figure 3 est une vue semblable à la figure 2, l'implant tibial étant représenté de trois quarts vue de dessous.
La figure 4 est une vue en plan de l'embase tibiale.
La figure 5 est une vue en coupe considérée selon la ligne 5.5 de la figure 4.
La figure 6 est une vue en coupe considérée selon la ligne 6.6 de la figure 4.

L'implant tibial comprend une embase tibiale (1) recevant un plateau tibial en polyéthylène (2) et une quille de stabilité et d'ancrage (3). Cet implant tibial coopère au niveau de son plateau (2), avec tout type d'implant fémoral (F). L'embase tibiale (1) est de forme asymétrique pour tenir compte de l'asymétrie existant entre les compartiments interne et externe de la tête tibiale.

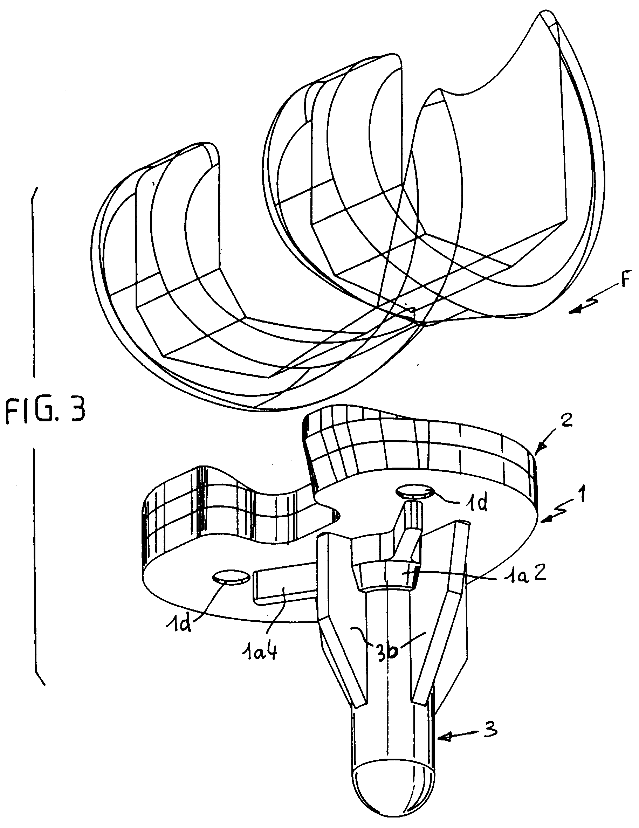

Selon l'invention, l'embase tibiale (1) présente une empreinte débouchante (1a) conformée pour permettre, à partir de sa face de dessus (1b), l'engagement, le centrage et le maintien en position de la quille (3). Cette empreinte (1a) est prolongée par un bossage (1a1) débordant de la face de dessous (1c) de l'embase tibiale (1). Ce bossage (1a1) est profilé pour faire office de quille de stabilité, afin d'assurer, si nécessaire, la fixation primaire de l'embase tibiale (1). Ce bossage (1a1) présente une extrémité tronconique (1a2). De même, ce bossage peut être prolongé latéralement par des nervures de rigidité (1a4) (figure 3).

L'empreinte (1a) présente un chambrage interne (1a3) pour le centrage et l'appui d'une tête (3a) formée à l'extrémité supérieure de la quille (3).

Dans l'exemple illustré, la quille (3) est formée par un corps cylindrique. Dans ces conditions, l'empreinte (1a) a une section correspondante. Par ailleurs, la tête (3a) de la quille (3) présente, à partir de sa périphérie, des fentes verticales (3a1). Cette tête, délimite un chambrage interne tronconique (3a2) débouchant dans un trou taraudé (1d), pour l'engagement d'une vis (4). Cette vis (4) a une tête tronconique de section complémentaire au chambrage (3a2), de sorte que lors de l'action de vissage dans la quille, la vis provoque l'expansion diamétrale de la tête (1a) de la quille, assurant ainsi sa liaison avec l'embase tibiale (1).

La quille (3) présente, de manière connue, en débordement de ses génératrices, des ailettes radiales de stabilité (3b). Dans ces conditions, l'empreinte débouchante (1a) de l'embase tibiale (1) présente des fentes (1a5) également débouchantes dont le nombre et l'orientation correspondent au nombre et à l'orientation des ailettes (3b) de la quille (3), en vue de leur engagement.

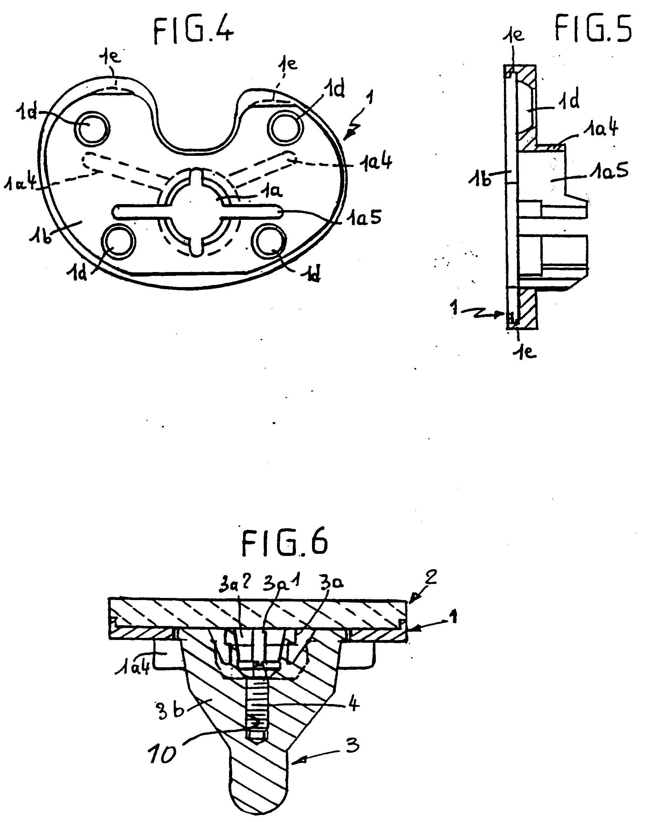

L'embase tibiale (1) présente également dans son épaisseur, des trous (1d) pour l'engagement de vis à os spongieux notamment, non représentées. Par exemple, comme le montre la figure 4, l'embase présente quatre trous (1d). De manière connue, ces trous sont conformés pour permettre de noyer les têtes de vis, en permettant éventuellement leur orientation angulaire.

Les caractéristiques techniques de l'embase tibiale (1), telles que définies, s'avèrent particulièrement avantageuses pour son impaction.

Dans un premier temps, le praticien positionne l'embase (1), non équipée de la quille, le bossage tronconique (1a1) facilitant la fixation primaire de ladite embase, permettant ainsi un partait appui péricortical.

Dans un deuxième temps, l'embase est fixée au moyen des vis à os spongieux, engagées dans les trous (1d). Il est alors possible d'introduire la quille au travers de l'empreinte (1a), laquelle fait également office de guide de perçage. Il suffit ensuite, de fixer la quille à l'embase, en provoquant l'expansion de la tête (3a) au moyen de la vis (4).

Il apparait donc que l'introduction de la quille (3), par le haut de l'embase (1), permet de la rendre solidaire de ladite embase, lorsque cette dernière est correctement impactée. Il en résulte que la quille n'influence pas de manière néfaste le positionnement de l'embase, de sorte que les appuis sont parfaitement respectés.

Après impaction de l'embase tibiale et de la quille (3) dans les conditions sus-indiquées, le praticien met en place le plateau en polyéthylène dans l'embase (1). A cet effet, de manière connue, l'embase (1) présente un rebord périphérique (1e) agencé pour la fixation par clipsage notamment, du plateau (2). Les formes et profils du plateau (2) ne sont pas décrits car ils peuvent présenter différentes formes de réalisation. Il en est de même en ce qui concerne l'implant fémoral (F).

Les éléments constitutifs de l'implant tibial, notamment de l'embase (1) et de la quille (3) sont réalisés en tout matériau et peuvent présenter tout type de traitement de surface.

On prévoit également, dans une forme de réalisation en variante non illustrée, d'agencer la tête de la quille et/ou l'empreinte débouchante (1a) de l'embase, de moyens complémentaires permettant l'orientation angulaire de ladite quille et son blocage en position. Par exemple, ces moyens peuvent être constitués par une tête sphérique, située au niveau de la tête (3a) et destinée à être engagée dans l'ouverture (1a) de l'embase tibiale de forme sphérique complémentaire.

Ces dispositions s'avèrent particulièrement avantageuses dans le cas de prothèses de reprises, pour lesquelles on utilise des longueurs de quilles importantes. En effet, si la quille est perpendiculaire à l'embase (1b) et que le chirurgien donne une pente postérieure à cette embase, la quille vient buter sur la corticale antérieure. Cette possibilité de réglage angulaire de la quille par rapport à l'embase, permet donc d'aligner ladite quille dans l'axe du canal médullaire tibial.

La quille (3) peut également être recouverte d'une matière céramique dense, non ostéoconductrice et à résorbtion programmable en modifiant la composition de ladite matière.

Les avantages ressortent bien de la description, en particulier on souligne et on rappelle :
- la mise en place de la quille, après impaction de l'embase tibiale , compte-tenu de son introduction à partir de la face de dessus de ladite embase, évitant ainsi de solliciter d'une manière néfaste, les appuis corticaux recherchés,
- la facilité d'impaction,
- la possibilité d'équiper l'embase de différentes formes de quilles adaptées au cas pathologique à traiter,
- l'orientation de la quille par rapport à l'embase tibiale en cas de prothèse de reprise notamment.

## Revendications

1. Implant tibial pour prothèse du genou comprenant une embase tibiale (1) munie d'une quille (3) d'ancrage et/ou de stabilité et un plateau tibial (2) adapté pour être disposé sur l'embase, caractérisé en ce que l'embase tibiale (1) présente une empreinte (1a) de forme et section déterminées, aptes à permettre à partir de la face de dessus de ladite embase (1) l'engagement, le centrage et le maintien en position, de ladite quille.

2. Implant selon la revendication 1, caractérisé en ce que l'empreinte (1a) est prolongée par un bossage (1a1) débordant de la face de dessous (1c) de l'embase tibiale (1), ledit bossage étant profilé pour faire office de quille d'ancrage.

3. Implant selon la revendication 1, caractérisé en ce que la quille rapportée (3) présente une tête épaulée (3a) apte à coopérer en appui dans un chambrage de l'empreinte (1a).

4. Implant selon la revendication 1, caractérisé en ce que l'empreinte (1a) présente des fentes débouchantes (1a5) dont le nombre et l'orientation correspondent au nombre et à l'orientation des ailettes (3b) de la quille (3), en vue de leur engagement.

5. Implant selon la revendication 1, caractérisé en ce que la tête (3a) de la quille (3) présente des agencements de fixation avec l'empreinte (1a).

6. Implant selon la revendication 5, caractérisé en ce que les agencements sont constitués par des lentes verticales (3a1) formées à partir de la périphérie de la tête (3a), en vue de son expansion diamètrale sous l'action d'une vis de serrage.

7. Implant selon la revendication 2, caractérisé en ce que le bossage (1a1) présente une extrémité tronconique (1a2).

8. Implant selon la revendication 3, caractérisé en ce que l'empreinte (1a) et la tête (3a) sont de forme complémentaire sphérique pour permettre l'orientation de la quille (3).

9. Implant selon la revendication 3, caractérisé en ce que l'empreinte (1a) et la tête (3a) sont de forme générale circulaire.

10. Implant selon la revendication 1, caractérisé en ce que l'embase tibiale (1) présente des trous (1d) pour le passage de vis de fixation.

11. Implant selon la revendication 1, caractérisé en ce que l'embase tibiale (1) est asymétrique et présente un rebord périphérique (1e) agencé pour la fixation du plateau tibial (2) en polyéthylène.

## Claims

1. Tibial implant for a knee prosthesis, comprising a tibial base (1) provided with an anchoring and/or stabilising pin (3) and a tibial plate (2) adapted to be arranged on the base, characterised in that the tibial base (1) has a hole (1a) of given shape and cross-section suitable for permitting the engagement, centring and maintenance in position of the pin, starting from the upper face of the base (1).

2. Implant according to claim 1, characterised in that the hole (1a) is extended by a boss (1a1) protecting from the lower face (1c) of the tibial base (1), the boss being shaped to act as an anchoring pin.

3. Implant according to claim 1, characterised in that the separate pin (3) has a supported head (3a) suitable for bearing cooperation in a recess of the hole (1a).

4. Implant according to claim 1, characterised in that the hole (1a) has open slots (1a5), the number and orientation of which correspond to the number and orientation of the fins (3b) of the pin (3) with a view to their engagement.

5. Implant according to claim 1, characterised in that the head (3a) of the pin (3) has means for securing to the hole (1a).

6. Implant according to claim 5, characterised in that the means are constituted by upright slots (3a1) formed starting from the periphery of the head (3a), with a view to its diametral expansion under the action of a tightening screw.

7. Implant according to claim 2, characterised in that the boss (1a1) has a frustoconical end (1a2).

8. Implant according to claim 3, characterized in that the hole (1a) and the head (3a) have a complementary spherical shape in order to permit the orientation of the pin (3).

9. Implant according to claim 3, characterised in that the hole (1a) and the head (3a) are of a generally circular shape.

10. Implant according to claim 1, characterised in that the tibial base (1) has openings (1d) for the passage of securing screws.

11. Implant according to claim 1, characterised in that the tibial base (1) is asymmetrical and has a peripheral flange (1e) adapted for securing the tibial plate (2) of polyethylene.

## Patentansprüche

1. Tibialimplantat für eine Kniegelenkprothese, mit einem Tibialsockel (1), der einen Verankerungs- und/oder Stabilisierungskiel (3) aufweist, und einer Tibialplatte (2), die auf dem Sockel anzuordnen ist, dadurch **gekennzeichnet**, daß der Tibialsockel (1) eins Aufnahme (1a) mit bestimmter Form und bestimmtem Querschnitt bildet, die es gestattet den Kiel von der Oberseite des Sockels (1) her einzustecken, zu zentrieren und in Position zu halten.

2. Implantat nach Anspruch 1, dadurch **gekennzeichnet**, daß die Aufnahme (1a) durch eine Nabe (1a1) verlängert ist, die von der Unterseite (1c) des Tibialsockels (1) vorsteht und profiliert ist, um als Verankerungskiel zudienen.

3. Implantat nach Anspruch 1, dadurch **gekennzeichnet**, daß der angesetzte Kiel (3) einen mit Schultern versehenen Kopf (3a) bildet, der dazu ausgebildet ist, in Anlage mit einer Einsenkung in der Aufnahme (1a) zusammenzuwirken.

4. Implantat nach Anspruch 1, dadurch **gekennzeichnet**, daß die Aufnahme (1a) durchgehende Schlitze (1a5) bildet, deren Anzahl und Orientierung der Anzahl und der Orientierung von Flügeln (3b) des Kiels (3) im Hinblick auf seinen Eingriff entsprechen.

5. Implantat nach Anspruch 1, dadurch **gekennzeichnet**, daß der Kopf (3a) des Kiels (3) Anordnungen zur Befestigung mit der Aufnahme (1a) aufweist.

6. Implantat nach Anspruch 5, dadurch **gekennzeichnet**, daß diese Anordnungen durch vertikale Schlitze (3a1) gebildet werden, die vom Umfang des Kopfes (3a) ausgehen, im Hinblick auf seine diametrale Expansion unter der Wirkung einer Spannschraube.

7. Implantat nach Anspruch 2, dadurch **gekennzeichnet**, daß die Nabe (1a1) ein kegelstumpfförmiges Ende (1a2) hat.

8. Implantat nach Anspruch 3, dadurch **gekennzeichnet**, daß die Aufnahme (1a) und der Kopf (3a) komplementäre sphärische Formen haben, um die Ausrichtung des Kiels (3) zu ermöglichen.

9. Implantat nach Anspruch 3, dadurch **gekennzeichnet**, daß die Aufnahme (1a) und der Kopf (3a) allgemein kreisförmig sind.

10. Implantat nach Anspruch 1, dadurch **gekennzeichnet**, daß der Tibialsockel (1) Löcher (1d) für den Druchtritt von Befestigungsschrauben hat.

11. Implantat nach Anspruch 1, dadurch **gekennzeichnet**, daß der Tibialsockel (1) asymmetrisch ist und am Umfang eine Randleiste (1e) aufweist, die zur Befestigung der Tibialplatte (2) aus Polyethylen ausgebildet ist.
